# EUROPEAN PATENT SPECIFICATION

(11) **EP 3 954 355 B1**
(45) Date of publication and mention of the grant of the patent: **12.06.2024**
(21) Application number: 21177555.6
(22) Date of filing: 03.06.2021
(51) Int. Cl.: A61J 1/20, A61M 39/26

(54) **CLOSED CONNECTOR WITH COMPRESSIBLE BUTTING JOINT TO ABSORB RESIDUAL LIQUID MEDICINE IN DISASSEMBLY**
GESCHLOSSENER VERBINDER MIT KOMPRIMIERBARER STOSSVERBINDUNG ZUR AUFNAHME VON FLÜSSIGER RESTMEDIZIN BEI DER DEMONTAGE
CONNECTEUR FERMÉ AVEC JOINT DE MISE EN BUTÉE COMPRESSIBLE POUR ABSORBER UN MÉDICAMENT LIQUIDE RÉSIDUEL LORS DU DÉMONTAGE

(30) Priority: 14.08.2020 TW 109127763
(43) Date of publication of application: 16.02.2022
(73) Proprietor: Tsai, Hsi-Chin, 238 New Taipei City (TW)
(72) Inventor: Tsai, Hsi-Chin, 238 New Taipei City (TW)
(74) Representative: Gee, Steven William

(56) References cited:
- CN-A- 104 096 283
- US-A1- 2019 282 797
- US-B1- 6 883 778

## Description

### BACKGROUND OF THE INVENTION

### 1. Field of the Invention

The present invention relates generally to a closed connector, and more particularly, to a closed connector with compressible butting joint to absorb residual liquid medicine in disassembly.

### 2. The Prior Arts

When a needleless syringe is replenishing liquid medicine, it is connected to a medicine bottle through a closed connector, and the medicine liquid in the medicine bottle is drawn into the syringe through the connector.

A conventional closed connector according to the preamble of claim 1 is disclosed in US2019/0282797 and includes a first connection assembly and a second connection assembly. The first connection assembly includes a first sleeve, an elastic body and a coupling member, and the second connection assembly includes a second sleeve, a connecting tube, a first elastic valve and a second elastic valve. A syringe or an infusion tube is detachably connected to the coupling member, and a medicine bottle or a human body catheter is detachably connected to the second sleeve. During the butting process of the first connection assembly and the second connection assembly, a first tube of the first sleeve compresses the first elastic valve, so that the first elastic valve flexes and deforms to open a slit, and the connecting tube subsequently passes through the slit of the first elastic valve and the opening of the first tube to enter the inside of the first tube, and the connecting tube pushes against the elastic body, causing the elastic body to flex and deform, and a perforation is exposed outside the first elastic valve and located inside the first tube. The connecting tube further compresses the second elastic valve, so that the second elastic valve flexes and deforms to open a slit. The first elastic valve continues to push the connecting tube to move downward until the connecting tube passes through the slit of the second elastic valve to enter a second tube of the second sleeve. Thereby, the inside of the first sleeve communicates with the inside of the second sleeve.

US Patent 6,883,778 and Chinese Patent Application 104 096 283 also disclose closed connectors for a similar purpose.

During the process of dispensing the pharmaceutical solution, the liquid medicine in the medicine bottle can flow into the syringe through the second tube, the connecting tube, the first tube, and the coupling member in sequence. During the procedure of injecting or delivering the liquid medicine, the liquid medicine in the syringe or the infusion tube can enter the human body through the coupling member, the first tube, the connecting tube, the second tube, and the human body catheter in sequence. After completing the process of preparing the pharmaceutical solution or the process of injecting or delivering the pharmaceutical solution, the first connection assembly and the second connection assembly are disassembled again, and there will be residues of the liquid medicine on the outer surface of the connecting tube.

Although in the process of disassembling the first connection assembly and the second connection assembly, the residual liquid medicine on the outer surface of the connecting tube will be slightly scraped off by the first tube, it is difficult to completely scrape clean, resulting in traces of liquid medicine residue on the surface of the connecting tube. It should be noted that some liquid medicines are cytotoxic (for example, chemotherapeutic drugs). Once the patient or caregiver's skin come in contact with the residual liquid medicine or the residual liquid medicine is inhaled in the lungs, it will affect the health of the patient or caregiver.

Furthermore, in some places with insufficient space, the infusion tube may be twisted, which will affect the smoothness of the liquid medicine delivery. The general processing is as follows: First, rotate the first connection assembly to make the first connection assembly loosened from the infusion tube; then, the twisted infusion tube is rearranged into a straight-line shape; finally, the infusion tube is detachably connected to the first connection assembly. Although the above process can solve the problem of twisting of the infusion tube, the operation is very troublesome, and there is a risk of leakage of the liquid medicine.

### SUMMARY OF THE INVENTION

A primary objective of the present invention is to provide a closed connector with compressible butting joint to absorb the residual liquid medicine in disassembly, which can effectively avoid the residual liquid medicine on the outer surface of the connecting tube, and reduce the risk of skin contact or inhalation by patients or nursing staff.

To achieve the foregoing objective, the present invention provides a closed connector with compressible butting joint to absorb residual liquid medicine in disassembly, including: a first connection assembly and a second connection assembly.

The first connection assembly includes a first sleeve, a coupling member, and a first elastic body. The first sleeve includes a first tube and a positioning groove. The first tube defines a first opening, and the inside of the first tube is provided with at least one supporting rib. The coupling member has a first end, a second end, and a blocking portion. The first end of the coupling member is arranged in the first sleeve, the second end of the coupling member is adapted for screwing on a syringe or an infusion tube, and the blocking portion is provided on the inside the first end of the coupling member. The first elastic body includes a circumferential portion and a sealing portion. The circumferential portion is positioned in the positioning groove and extends into the first tube, with an outer side wall abutting against the at least one supporting rib, and has an accommodating space and at least one suction hole. The accommodating space penetrates through the top end of the circumferential portion, and the at least one suction hole is connected between the accommodating space and the inside of the first tube. The first end of the coupling member presses on the top end of the circumferential portion of the first elastic body, the blocking portion abutting against the top end of the circumference portion and closing an opening of the accommodating space, and the sealing portion is provided in the first tube to close the first opening.

The second connection assembly includes a second sleeve and a communication tube. The second sleeve has a second opening and is adapted to detachably connect to a medicine bottle or a human body catheter. The communication tube is arranged in the second sleeve, having a closed top end, and is disposed with at least one perforation.

Wherein, during a docking process of the first connection assembly and the second connection assembly, the communication tube pushes and compresses the sealing portion, and the sealing portion compresses the circumferential portion, so that the air in the accommodating space enters the inside of the first tube through the at least one suction hole, the at least one suction hole is deformed and closed to make the accommodating space in a vacuum state. The communication tube passes through the first opening into the inside of the first tube. The at least one perforation is located in the first tube, so that the inside of the first sleeve communicates with the inside of the second sleeve.

Wherein, during a process of disassembling the first connection assembly and the second connection assembly, the communication tube is separated from the first tube.

Wherein, in a process of the communication tube separating from the first tube, the first tube contacts the outer surface of the communication tube. In a process of the first elastic body restoring to its original shape by elastic force, the circumferential portion is restored by pushing against the sealing portion with elastic force to close the first opening, and the at least one suction hole is restored to its open position, the air inside the first tube entering the accommodating space through the at least one suction hole to generate negative pressure, and the liquid medicine on the outer surface of the communication tube is caused to enter the first tube through a scraping action of the first tube and a suction action of negative pressure, and the liquid medicine inside the first tube further passes through the at least one suction hole to be collected in the accommodating space through the suction action of the negative pressure.

The effect of the present invention is that the present invention can effectively prevent the liquid medicine from remaining on the outer surface of the communication tube, and reduce the risk of skin contact or inhalation by patients or caregivers.

### BRIEF DESCRIPTION OF THE DRAWINGS

The present invention will be apparent to those skilled in the art by reading the following detailed description of a preferred embodiment thereof, with reference to the attached drawings, in which:
Fig. 1 is a cross-sectional view illustrating the first embodiment according to the present invention;
Fig. 2 is a cross-sectional view along the A-A line of Fig. 1;
Fig. 3 is a cross-sectional view illustrating the first sleeve of the first embodiment according to the present invention;
Fig. 4 is a cross-sectional view illustrating the first elastic body of the first embodiment according to the present invention;
Fig. 5 is a schematic view illustrating the use of the first embodiment according to the present invention;
Fig. 6 is a cross-sectional view illustrating the second embodiment according to the present invention; and
Fig. 7 is a cross-sectional view illustrating the third embodiment according to the present invention.

### DETAILED DESCRIPTION OF THE PREFERRED EMBODIMENT

The accompanying drawings are included to provide a further understanding of the invention, and are incorporated in and constitute a part of this specification. The drawings illustrate embodiments of the invention and, together with the description, serve to explain the principles of the invention.

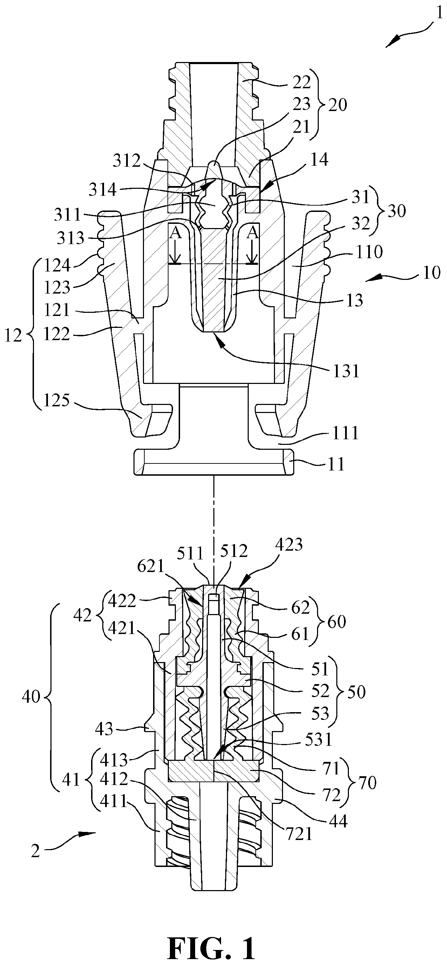

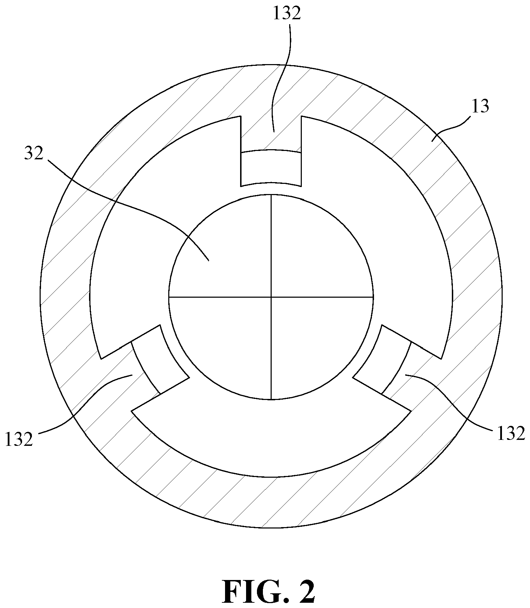

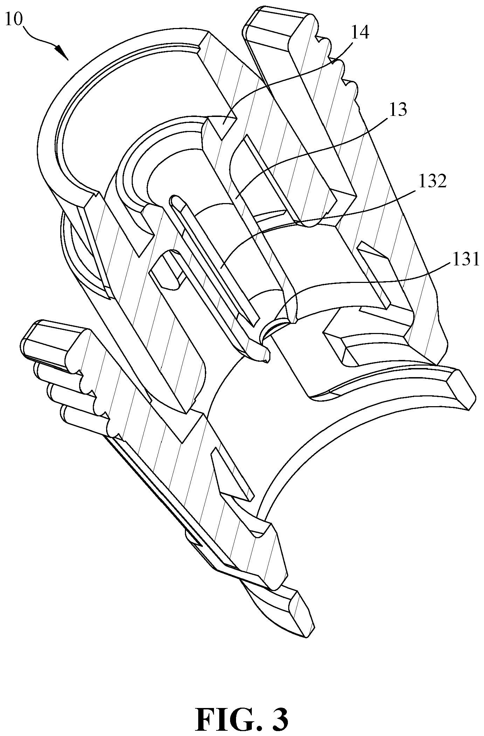

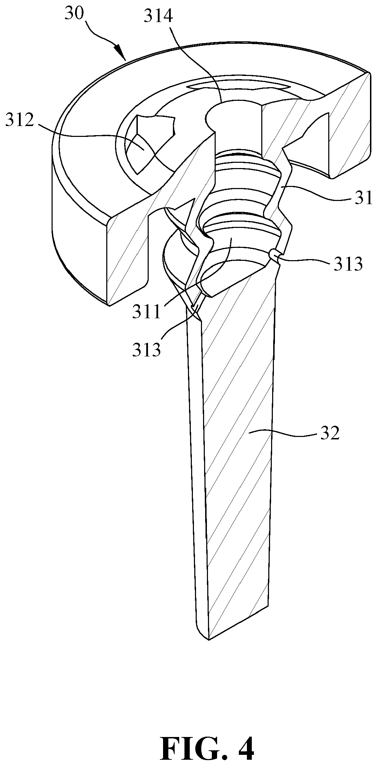

Referring to Figs. 1-4, the present invention provides a closed connector with compressible butting joint to absorb residual liquid medicine in disassembly, including: a first connection assembly 1 and a second connection assembly 2.

The first connection assembly 1 includes a first sleeve 10, a coupling member 20, and a first elastic body 30.

The first sleeve 10 includes a sleeve member 11, two clamping portions 12, a first tube 13 and a positioning groove 14. The sleeve member 11 defines two perforations 111. The perforations 111 penetrate through two opposite sides of the sleeve member 11 and communicate with the inside of the sleeve member 11. Each clamping portion 12 includes a connecting portion 121 and a clamping arm 122. The connecting portions 121 respectively protrude on two opposite sides of the sleeve member 11 and are located above the perforations 111. The clamping arms 122 are respectively pivoted on the outside of the connecting parts 121. One end of each clamping arm 122 is a handle 123, and the outer surface of each handle 123 is provided with a plurality of anti-slip strips 124. When the user's fingers press on each handle 123, the anti-slip strips 124 can provide excellent anti-slip effect. A hook 125 is formed at the other end of each clamping arm 122, and the hooks 125 are respectively located in the perforations 111. The first tube 13 defines a first opening 131, and the inside of the first tube 13 has three supporting ribs 132.

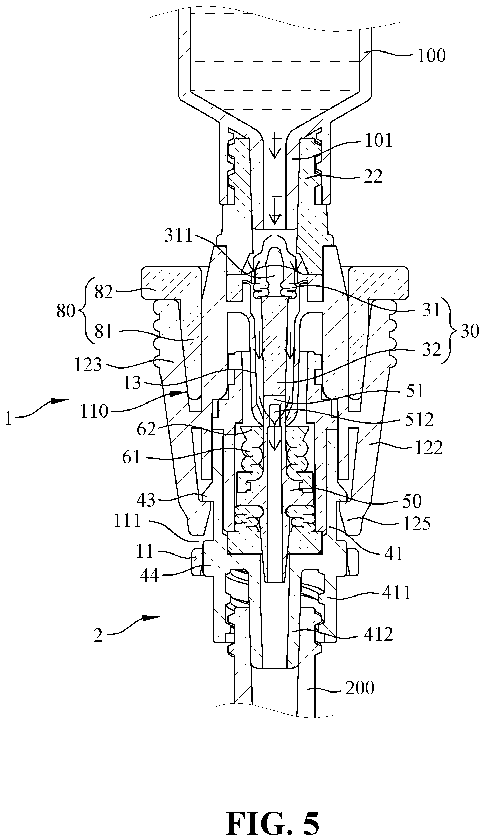

The coupling member 20 has a first end 21, a second end 22, and a blocking portion 23. The first end 21 is provided inside the sleeve member 11, and the second end 22 is used for screwing on a syringe 100 (see Fig. 5) or an infusion tube (not shown). As shown in Fig. 5, a catheter 101 of the syringe 100 or the infusion tube is inserted into the second end 22 and communicates with the inside of the coupling member 20. The blocking portion 23 is provided inside the first end 21.

The first elastic body 30 includes a circumferential portion 31 and a sealing portion 32. The circumferential portion 31 is positioned inside the positioning groove 14 and extends to the inside of the first tube 13. The outer side wall abuts against the supporting ribs 132 and has an accommodating space 311, a plurality of perforations 312 and a plurality of suction holes 313. The accommodating space 311 penetrates the top end of the circumferential portion 31, the perforations 312 communicate between the inside of the first end 21 and the inside of the first tube 13, and the suction holes 313 communicate between the accommodating space 311 and the inside of the first tube 13. The first end 21 presses on the top end of the circumferential portion 31, and the blocking portion 23 abuts against the top end of the circumferential portion 31 and closes an opening 314 of the accommodating space 311. The sealing portion 32 is provided in the first tube 13 to close the first opening 131.

The second connection assembly 2 includes a second sleeve 40, a communication tube 50, a first elastic valve 60, and a second elastic valve 70.

The second sleeve 40 includes a first cylinder 41, a second cylinder 42, a protrusion 43, and a stabilizing structure 44. The first cylinder 41 has a screw connection portion 411, a second tube 412, and a cylinder portion 413. The screw connection portion 411 has an internal thread. The second tube 412 extends into the screw connection portion 411, and the cylinder portion 413 is disposed above the screw connection portion 411. The second cylinder 42 has a first end 421 and a second end 422. The first end 421 is disposed inside the cylinder portion 413, and the second end 422 is located outside the cylinder portion 413 and is a screw connection portion having an external thread, and is disposed with a second opening 423. The protrusion 43 is provided around the outer side of the cylindrical portion 413. The stabilizing structure 44 is arranged around the outer side of the first cylinder 41 and located below the protrusion 43. As shown in Fig. 5, one end of a medicine bottle 200 or a human body catheter (not shown) is screwed on the screw connection portion 411, and the second tube 412 is inserted into the inside of the medicine bottle 200 or one end of the human body catheter, so that the inside of the second tube 412 communicates with the inside of the medicine bottle 200 or the human body catheter.

The communication tube 50 is arranged inside the second cylinder 42 and has an upper pillar portion 51, a large-diameter portion 52, and a lower pillar portion 53. The upper pillar portion 51 is disposed above the large-diameter portion 52, with a closed top end 511, and provided with two perforations 512; the perforations 512 respectively penetrate through two opposite sides of a side wall of the upper pillar portion 51, and the perforations 512 are located close to the closed top end 511 of the upper pillar portion 51 and communicate with the inside of the upper pillar portion 51. The lower pillar portion 53 is disposed below the large-diameter portion 52, with an open bottom end 531. The inside of the upper pillar portion 51 and the inside of the large-diameter portion 52 communicate with the inside of the lower pillar portion 53.

The first elastic valve 60 is disposed inside the second cylinder 42 and has a circumferential portion 61 and a sealing portion 62. The circumferential portion 61 is arranged around the outer side of the upper pillar portion 51, with a bottom end abutting against the large-diameter portion 52. The sealing portion 62 is disposed at the top end of the circumferential portion 61, and the sealing portion 62 defines a slit 621. The circumferential portion 61 can push against the sealing portion 62 by its elastic force, so that the sealing portion 62 closes the second opening 423.

The second elastic valve 70 is disposed inside the second cylinder 42 and has a circumferential portion 71 and a sealing portion 72. The circumferential portion 71 is arranged around the outer side of the lower pillar portion 53, with top end abutting against the large-diameter portion 52. The circumferential portion 71 can push against the large-diameter portion 52 by its elastic force, and the large-diameter portion 52 further pushes against the upper pillar portion 51 so that the upper pillar portion 51 is located in the slit 621; the closed top end 511 of the upper pillar portion 51 and the top end of the slit 621 are aligned, and the perforations 512 are closed by the sealing portion 62. The sealing portion 72 is provided at the bottom end of the circumferential portion 71 and abuts against the inner side of the cylinder portion 413, and the bottom end of the first end portion 421 presses the top end of the sealing portion 72. The sealing portion 72 defines a slit 721, the slit 721 is aligned with the inside of the second tube 412 and the inside of the lower pillar portion 53, and the bottom end of the lower pillar portion 53 abuts against the top end of the sealing portion 72. The sealing portion 72 keeps the slit 721 closed by its elastic force, so that the sealing portion 72 closes the inside of the lower pillar portion 53.

Before the process of dispensing the pharmaceutical solution, the first connection assembly 1 is fixed to the syringe 100, and the second connection assembly 2 is fixed to the medicine bottle 200. Because the slit 721 is kept closed, the liquid medicine in the medicine bottle 200 only enters the second tube 412 and does not enter the inside of the communication tube 50.

Also, before the process of injecting or delivering the liquid medicine, the first connection assembly 1 is fixed to the syringe 100 or the infusion tube, and the second connection assembly 2 is fixed to the human body catheter. Because the sealing portion 32 closes the first opening 131, the medical liquid in the syringe 100 or the infusion tube cannot flow out through the first opening 131.

Referring to Fig. 5, during the docking process of the first connection assembly 1 and the second connection assembly 2, the upper pillar portion 51 pushes against the sealing portion 32, the sealing portion 32 compresses the circumferential portion 31, and the air in the accommodating space 311 enters the inside of the first tube 13 through the suction holes 313, and the suction holes 313 are deformed and closed, so that the accommodating space 311 is in a vacuum state. The upper pillar portion 51 passes through the first opening 131 to enter the inside of the first tube 13, and the first tube 13 pushes against the sealing portion 62. The perforations 512 of the upper pillar portion 51 are exposed outside the sealing portion 62 and located inside the first tube 13. The circumferential portion 61 pushes against the large-diameter portion 52, and the large-diameter portion 52 compresses the circumferential portion 71 so that the circumferential portion 71 flexes and deforms. At this point, the circumferential portion 71 pushes against the communication tube 50 to move this downward, and the lower pillar portion 53 expands the slit 721 and then passes through the slit 721 to enter the inside of the second tube 412. After the first cylinder 41 enters the sleeve member 11, the stabilizing structure 44 abuts against the inner side wall of the sleeve member 11. Thereby, the inside of the coupling member 20, the first tube 13, the communication tube 50, and the inside of the second tube 412 communicate with one another. The protrusion 43 moves into the perforations 111, and the hooks 125 hook the bottom end of the protrusion 43.

Preferably, as shown in Fig. 1, a groove 110 is formed between the handle 123 and the sleeve member 11; as shown in Fig. 5, the closed connector further includes a safety ring 80, and the safety ring 80 includes a sleeve portion 81 and a ring portion 82, the sleeve portion 81 is movably sleeved on the sleeve member 11, and the ring portion 82 is provided on the top end of the sleeve portion 81. After the first connection assembly 1 and the second connection assembly 2 are docked, the sleeve portion 81 moves along the sleeve member 11 into the groove 110, so that the sleeve portion 81 is clamped inside the groove 110, and the ring portion 82 abuts against the top of the handle 123. In case the patient accidentally presses the handles 123, the sleeve portion 81 can block the handles 123 and prevent the other end of the clamping arms 122 from pivoting, and the hooks 125 can keep hooking the bottom end of the protrusions 43, so that the first connection assembly 1 and the second connection assembly 2 are kept in the docking state, and the first connection assembly 1 and the second connection assembly 2 will not be disassembled.

During the dispensing process, the liquid medicine in the medicine bottle 200 can flow into the syringe 100 through the second tube 412, the communication tube 50, the first tube 13 and the inside of the coupling member 20 in sequence.

When injecting or delivering liquid medicine, the liquid medicine in the syringe 100 or the infusion tube can sequentially pass through the inside of the coupling member 20, the first tube 13, the communication tube 50, the second tube 412, and the human body catheter, and into the human body.

If the second end 22 is fixed to the infusion tube and the infusion tube is severely twisted and deformed for some reason, the protrusion 43 can be arbitrarily rotated in a first direction or a second direction along the hooks 125, the first connection assembly 1 can rotate arbitrarily along the first direction or the second direction relative to the second connection assembly 2, so that the infusion tube can be untwisted into a straight line without the leakage of the liquid medicine; therefore, the liquid medicine in the infusion tube can keep flowing smoothly.

After completing the process of dispensing, injecting or delivering the liquid medicine, the first connection assembly 1 and the second connection assembly 2 are disassembled again, and the outer surface of a part of the upper pillar portion 51 located inside the first tube 13 will be liquid medicine residue.

During the preliminary disassembly process of the first connection assembly 1 and the second connection assembly 2, the user's fingers grasp the ring portion 82 and move this upward, so that the sleeve portion 81 moves along the sleeve member 11 and escapes from the groove 110. The handles 123 are no longer blocked by the sleeve portion 81 and can be pressed. The other end of the clamping arms 122 can pivot freely. By pressing the handles 123, the other end of the clamping arms 122 pivots outwards, the hooks 125 disengage from the protrusion 43. The circumferential portion 71 pushes against the large-diameter portion 52 by its elastic force, so that the communication tube 50 and the first elastic valve 60 move upward, and the lower pillar portion 53 moves upwards away from the second tube 412 and returns to the inside of the circumferential portion 71; the sealing portion 72 completely closes the slit 721 by its elastic force, so that the sealing portion 72 seals the inside of the lower pillar portion 53 and blocks the inside of the communication tube 50 and the second tube 412, increases the sealing effect of the inside of the second connection assembly 2 and prevents the liquid medicine from flowing from the medicine bottle 200 into the communication tube 50. The circumferential portion 61 pushes the sealing portion 62 upward by its elastic force, causing the sealing portion 62 to move upward along the outer side of the upper pillar portion 51, and the sealing portion 62 pushes against the first tube 13 by its elastic force to drive the first connection assembly 1 to move so that the upper pillar portion 51 detaches from the first tube 13 and returns to the sealing portion 62.

What is important is that the sealing portion 62 will continue to abut against the first tube 13 during the upward movement, and the upper pillar portion 51 can always remain in the open slit 621 and the first tube during the upward movement. Therefore, the distance between the inside of the first end 21 and the inside of the second end 422 will not increase.

During the further disassembly process of the first connection assembly 1 and the second connection assembly 2, the sealing portion 62 continues to move upwards and covers the perforations 512, thus isolating the inside of the first tube 13 and the inside of the communication tube 50, and the liquid medicine will no longer flow into the first tube 13 or the communication tube 50 through the perforations 512. At this point, the upper pillar portion 51 maintains the state of expanding the slit 621, and the perforations 512 are closed by the sealing portion 62.

During the final disassembly of the first connection assembly 1 and the second connection assembly 2, the upper pillar portion 51 gradually passes through the first opening 131 to escape the first tube 13 and return to the slit 621, and the sealing portion 62 seals the perforations 512. In the process of the upper pillar portion 51 separating from the first tube 13, the first tube 13 will contact the outer surface of the upper pillar portion 51. During the process of the first elastic body 30 restoring to its original shape by elastic force, the circumferential portion 31 is pushed against the sealing portion 32 by its elastic force to restore its shape, and the sealing portion 32 closes the first opening 131, so the liquid medicine will not flow through the first opening 131 back to the first tube 13. The suction holes 313 return to the original shape and open, and the air inside the first tube 13 enters the accommodating space 311 through the suction holes 313 to generate negative pressure. The liquid medicine on the outer surface of the upper pillar portion 51 enters the inside of the first tube 13 by the scraping effect of the first tube 13 and the suction effect of the negative pressure, and the liquid medicine inside the first tube 13 further passes through the suction holes 313 to be collected in the accommodating space 311 via the suction effect of the negative pressure.

Therefore, the outer surface of the upper pillar portion 51 has no liquid medicine residue at all, and the liquid medicine collected in the accommodating space 311 will not flow out from the first opening 131, which reduces the risk of skin contact or inhalation by patients or care-givers.

Furthermore, during the installation process of the first connection assembly 1, the circumferential portion 31 is positioned in the positioning groove 14, and the first end 21 is inserted into the sleeve member 11 and presses the top end of the circumferential portion 31. The installation is easy and can also prevent the first elastic body 30 from escaping from the first sleeve 10.

In addition, because the stabilizing structure 44 is ring-shaped and abuts against the inner side wall of the sleeve member 11, the second sleeve 40 will not rattle inside the sleeve member 11.

Moreover, during the docking process of the first connecting assembly 1 and the second connecting assembly 2, the supporting ribs 132 can prevent the sealing portion 32 from flexing and deforming, so that the sealing portion 32 can maintain the axial movement to compress the circumferential portion 31 to ensure that the air in the accommodating space 311 is discharged and the suction holes 313 are deformed and closed.

Moreover, during the docking process of the first connection assembly 1 and the second connection assembly 2, the blocking portion 23 can prevent the middle of the circumferential portion 31 from moving upward and deforming, so that the circumferential portion 31 can be more reliably compressed by the sealing portion 32. The air in the accommodating space 311 can be completely evacuated to ensure the accommodating space 311 maintains a vacuum state.

Moreover, the blocking portion 23 can also prevent the liquid medicine from flowing into the accommodating space 311 through the opening 314, and ensure that the accommodating space 311 maintains a vacuum state.

It is worth noting that because the second end 422 has an external thread, the user can also screw the second end 422 to a syringe (not shown). In other words, in addition to being able to interface with the first connection assembly 1, the second connection assembly 2 can also be interfaced with a syringe.

In addition, the second end 22 is substantially equivalent to the female connector of the traditional Luer connector. When used in combination with the clamping portion 12, the first connection assembly 1 has the function of the female connector of the traditional Luer connector and the clamping function at the same time. Therefore, the first connection assembly 1 can be combined with the syringe 100 or the infusion tube having the male connector of the traditional Luer connector and the second connection assembly 2 at the same time.

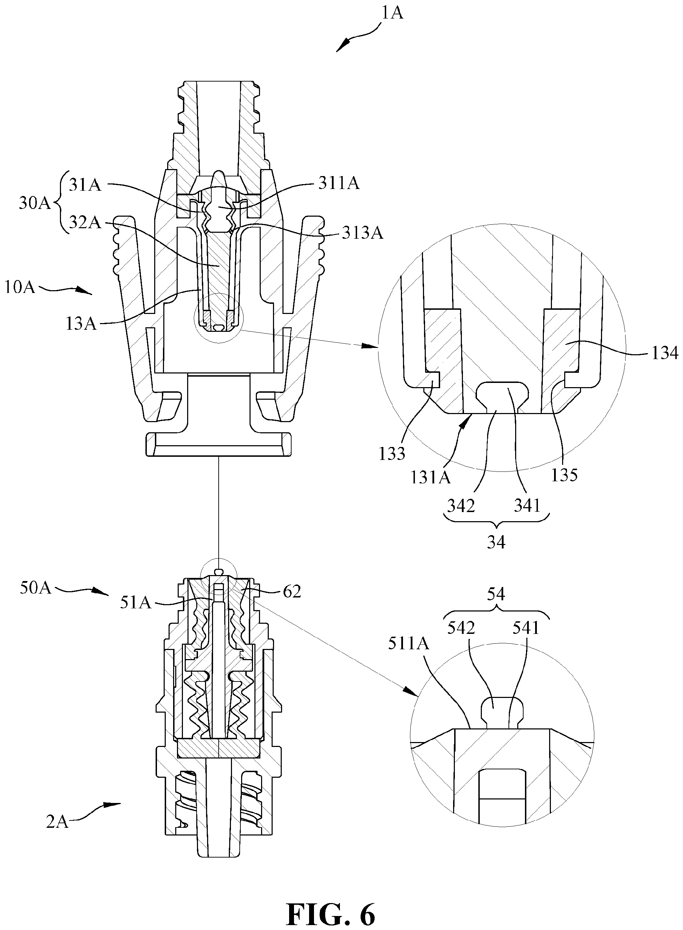

Referring to Fig. 6, the difference between the second embodiment and the first embodiment is: First, the bottom end of the first tube 13A forms an engaging portion 133, and the first tube 13A is provided with a closed collar 134. The closed collar 134 is made of a soft material and has a first opening 131A, and a groove 135 is recessed on the outer side. The engaging portion 133 is locked in the groove 135, and the sealing portion 32A is provided in the first tube 13A and the closed collar 134 to close the first opening 131A. Second, the first elastic body 30A has a first buckle portion 34, the first buckle portion 34 is provided in the sealing portion 32A, the first buckle portion 34 includes a recessed hole 341 and a notch 342; the recessed hole 341 is located inside the sealing portion 32A, the notch 342 is located at the bottom end of the sealing portion 32A, and the diameter of the notch 342 is smaller than the maximum diameter of the recessed hole 341. Third, the communication tube 50A includes a second buckle portion 54, the second buckle portion 54 includes a neck 541 and a head 542; the neck 541 is protruding from the closed end 511A, and the head 542 is disposed at one end of the neck 541; the diameter of the neck 541 is smaller than the maximum diameter of the head 542; the size and shape of the head 542 correspond to the size and shape of the recessed hole 341, and the size and shape of the neck 541 correspond to the size and shape of the notch 342. Except for the above, the remaining structure of the second embodiment is the same as that of the first embodiment.

Before performing the process of injecting or delivering the liquid medicine, the difference between the second embodiment and the first embodiment is that because the sealing portion 32A closes the first opening 131A, the liquid medicine in the syringe 100 or the infusion tube cannot pass through the first opening 131A to flow out.

During the docking process of the first connection assembly 1A and the second connection assembly 2A, the difference between the second embodiment and the first embodiment is: First, the head 542 enters the recessed hole 341, and the neck 541 is located in the notch 342, the second buckle portion 54 is buckled to the first buckle portion 34. Second, the upper pillar portion 51A pushes against the sealing portion 32A, so that the sealing portion 32A is separated from the closed collar 134. Third, the upper pillar portion 51A passes through the first opening 131A and the closed collar 134 to enter the inside of the first tube 13A. Fourth, the closed collar 134 is pushed against the sealing portion 62. Except for the above, the remaining docking process of the second embodiment is the same as that of the first embodiment.

During the disassembly process of the first connection assembly 1A and the second connection assembly 2A, the difference between the second embodiment and the first embodiment is: First, the sealing portion 62 pushes against the closed collar 134 by elastic force; the first connection assembly 1A is driven to move, so that the upper pillar portion 51A is separated from the closed collar 134 and returns to the sealing portion 62. Second, the second buckle portion 54 is pulled out from the first buckle portion 34, so that the first buckle portion 34 is separated from the second buckle portion 54. Third, when the upper pillar portion 51A is separated from the closed collar 134, the closed collar 134 will contact the outer surface of the upper pillar portion 51A. Fourth, in the process of an elastic body 30A restoring to original shape by elastic force, the circumferential portion 31A is pushed against the sealing portion 32A by elastic force to restore, so that the sealing portion 32A enters the closed collar 134 to close the first opening 131A. Fifth, the liquid medicine on the outer surface of the upper pillar portion 51A enters the inside of the first tube 13A through the scraping effect of the closed collar 134 and the suction effect of the negative pressure, and the liquid medicine inside the first tube 13A is further absorbed by the negative pressure to pass through the suction holes 313A to be collected in the accommodating space 311A. Except for the above, the remaining disassembly process of the second embodiment is the same as that of the first embodiment.

As such, the outer surface of the upper pillar portion 51A has no liquid medicine residue at all, and the liquid medicine collected in the accommodating space 311A will not flow out from the first opening 131A; therefore, the present invention reduces the risk of skin contact or inhalation of residual liquid medicine by the patients or care-givers.

Furthermore, since the second buckle portion 54 is buckled to the first buckle portion 34, during the docking and disassembly process of the first connection assembly 1A and the second connection assembly 2A, the upper pillar portion 51A and the sealing portion 32A can pull each other.

Moreover, the engaging portion 133 is clamped in the groove 135 to fix the closed collar 134 and prevent the closed collar 134 from being separated from the first sleeve 10A.

Moreover, the engaging portion 133 can allow the closed collar 134 to be more closely attached to the outer surface of the sealing portion 32A, thus, completely preventing the liquid medicine from flowing out through the gap between the closed collar 134 and the sealing portion 32A.

Moreover, since the bottom end of the closed collar 134 is exposed outside the first tube 13A, the user can easily sterilize the outer surface of the bottom end of the closed collar 134, which improves the safety of use.

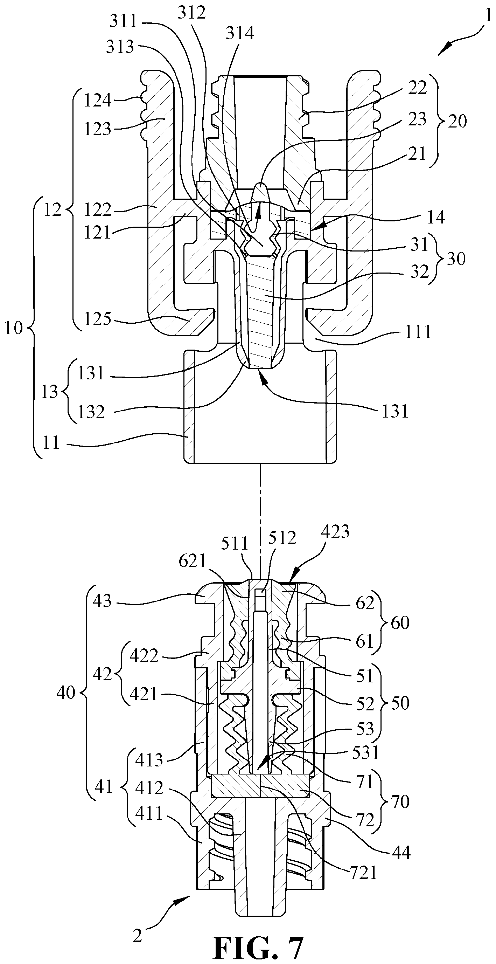

Referring to Fig. 7, the difference between the third embodiment and the first embodiment is that the structures of the first sleeve 10 and the second sleeve 20 are slightly different. Except for the above, the remaining technical features of the third embodiment are completely the same as those of the first embodiment.

Although the present invention has been described with reference to the preferred embodiments thereof, it is apparent to those skilled in the art that a variety of modifications and changes may be made without departing from the scope of the present invention which is defined by the appended claims.

## Claims

1. A closed connector with compressible butting joint to absorb residual liquid medicine in disassembly, comprising:
a first connection assembly (1), comprising:
a first sleeve (10), comprising: a first tube (13) and a positioning groove (14), the first tube (13) defining a first opening (131);
a coupling member (20), having a first end (21), and a second end (22), and the first end (21) of the coupling member (20) being arranged inside the first sleeve (10), the second end (22) of the coupling member (20) being adapted for screwing on a syringe (100) or an infusion tube; and
a first elastic body (30), comprising: a circumferential portion (31) and a sealing portion (32); the circumferential portion (31) being positioned in the positioning groove (14) and extending into the first tube (13); the first end (21) of the coupling member (20) pressing on the top end of the circumferential portion (31) of the first elastic body (30); the sealing portion (32) being provided in the first tube (13) to close the first opening (131); and
a second connection assembly (2), including:
a second sleeve (40) with a second opening (423) and adapted for detachably connecting to a medicine bottle (200) or a human body catheter; and
a communication tube (50), arranged in the second sleeve (40), having a closed top end (511), and disposed with at least one perforation (512);
**characterised in that** the coupling member (20) has a blocking portion (23), and the blocking portion (23) is provided on the inside of the first end (21) of the coupling member (20); the circumferential portion (31) has an accommodating space (311) and at least one suction hole (313); the accommodating space (311) penetrating through the top end of the circumferential portion (31), the at least one suction hole (313) being connected between the accommodating space (311) and the inside of the first tube (13); the inside of the first tube (13) has at least one supporting rib (132), **in that** an outer side wall of the first elastic body abuts against the at least one supporting rib (132), **in that** the blocking portion (23) of the coupling member abuts against the top end of the circumferential portion (31) and closes an opening (314) of the accommodating space (311);
**in that**, during a docking process of the first connection assembly (1) and the second connection assembly (2), the communication tube (50) pushes and compresses the sealing portion (32), and the sealing portion (32) compresses the circumferential portion (31), so that the air in the accommodating space (311) enters the inside of the first tube (13) through the at least one suction hole (313), and the at least one suction hole (313) is deformed and closed to make the accommodating space (311) in a vacuum state; the communication tube (50) passes through the first opening (131) into the inside of the first tube (13), and the at least one perforation (512) is located in the first tube (13), so that the inside of the first sleeve (10) communicates with the inside of the second sleeve (40);
**in that**, during a disassembly process of the first connection assembly (1) and the second connection assembly (2), the communication tube (50) is separated from the first tube (13); and
**in that**, in a process of the communicating tube (50) separating from the first tube (13), the first tube (13) contacts the outer surface of the communication tube (50), and in a process of the first elastic body (30) restoring to its original shape by elastic force, the circumferential portion (31) is restored by pushing against the sealing portion (32) with elastic force to close the first opening (131), and the at least one suction hole (313) is restored to its open position, the air inside the first tube (13) enters the accommodating space (311) through the at least one suction hole (313) to generate a negative pressure, and the liquid medicine on the outer surface of the communication tube (50) is caused to enter the first tube (13) through a scraping action of the first tube (13) and a suction action of negative pressure, and the liquid medicine inside the first tube (13) further passes through the at least one suction hole (313) to be collected in the accommodating space (311) through the suction action of the negative pressure.

2. The closed connector according to claim 1, wherein the first tube (13A) has a bottom end which forms an engaging portion (133), and the first tube (13A) is provided with a closed collar (134), the closed collar (134) being made of soft material and disposed with the first opening (131A) and a groove (135) recessed on the outer side; the engaging portion (133) is clamped in the groove (135), and the sealing portion (32A) is provided in the closed collar (134);
wherein, the first elastic body (30A) has a first buckle portion (34), the first buckle portion (34) is disposed on the sealing portion (32A), and the first buckle portion (34) comprises a recessed hole (341) and a notch (342); the recessed hole (341) is located inside the sealing portion (32A), the notch (342) is located at the bottom end of the sealing portion (32A), and the diameter of the gap (342) is smaller than the maximum diameter of the recessed hole (341);
wherein, the communication tube (50A) comprises a second buckle portion (54), the second buckle portion (54) comprises a neck (541) and a head (542); the neck (541) is disposed in a protruding manner at the closed end (511), the head (542) is disposed at one end of the neck (541), the diameter of the neck (541) is smaller than the maximum diameter of the head (542), the size and shape of the head (542) correspond to the size and shape of the recessed hole (341), and the size and shape of the neck (541) correspond to the size and shape of the notch (342);
wherein, in a docking process of the first connection assembly (1A) and the second connection assembly (2A), the head (542) enters the recessed hole (341), and the neck (541) is located in the notch (342), so that the second buckle portion (54) is buckled to the first buckle portion (34); the sealing part (32A) is separated from the closed collar (134), the communicating tube (50A) passes through the closed collar (134) into the first tube (13A);
wherein, in a disassembly process of the first connection assembly (1A) and the second connection assembly (2A), the communication tube (50A) is separated from the closed collar (134), and finally the second buckle portion (54) is pulled out from the first buckle portion (34), so that the first buckle portion (34) and the second buckle portion (54) are separated; and
wherein, in a process of the communication tube (50A) being separated from the closed collar (134), the closed collar (134) contacts the outer surface of the communication tube (50A), in a process of the first elastic body (30A) restoring to original shape by elastic force, the sealing portion (32A) enters the closed collar (134), and the liquid medicine on the outer surface of the communication tube (50A) enters the first tube (13A) through the scraping effect of the closed collar (134).

3. The closed connector according to claim 1 or 2, wherein the first sleeve (10) comprises at least one clamping portion (12), and the second sleeve (40) comprises a protrusion (43); wherein, during the docking process of the first connection assembly (1) and the second
connection assembly (2), the at least one clamping portion (12) clamps the protrusion (43);
wherein, by the protrusion (43) rotating arbitrarily in a first direction or a second direction along the at least one clamping portion (12), the first connection assembly (1) can move along the second connection assembly (2) to rotate arbitrarily in the first direction and the second direction; and
wherein, during the disassembly process of the first connection assembly (1) and the second connection assembly (2), the at least one clamping portion (12) is separated from the protrusion (43).

4. The closed connector according to claim 3, wherein the first sleeve (10) comprises a sleeve member (11), and the sleeve member (11) defines at least one perforation (111), the at least one perforation (111) penetrates a side wall of the sleeve member (11) and communicates with the inside of the sleeve member (11); the at least one clamping portion (12) comprises a connecting portion (121) and a clamping arm (122), the connecting portion (121) is protrudingly arranged on the outside of the sleeve member (11) and located above the at least one perforation (111), and the clamping arm (122) is pivoted on the outside of the connecting portion (121), one end of the clamping arm (122) is a handle (123), and the other end of the clamping arm (122) forms a hook (125), and the hook (125) is located at the at least one perforation (111); the protrusion (43) is arranged around the outside of the second sleeve (40);
wherein, in the docking process of the first connection assembly (1) and the second connection assembly (2), the protrusion (43) moves into the at least one perforation (111), and the hook (125) hooks the bottom end of the protrusion (43);
wherein, by the protrusion (43) being arbitrarily rotated in the first direction and the second direction along the hook (125), the first connection assembly (1) can be arbitrarily rotated along the first direction and the second direction relative to the second connection assembly (2); and
wherein in the process of disassembling the first connection assembly (1) and the second connection assembly (2), by pressing the handle (123), the other end of the clamping arm (122) pivots outward, the hook (125) is detached from the protrusion (43).

5. The closed connector according to claim 4, wherein a groove (110) is formed between the handle (123) and the sleeve member (11), and the closed connector further comprises a safety ring (80), and the safety ring (80) comprises a sleeve portion (81) and a ring portion (82); the sleeve portion (81) is movably sleeved on the sleeve member (11), and the ring portion (82) is arranged at the top end of the sleeve portion (81);
wherein, after the first connection assembly (1) and the second connection assembly (2) are docked in use, the sleeve portion (81) moves along the sleeve member (11) into the groove (110) so that the sleeve portion (81) is clamped in the groove (110) to block the handle (123) and prevent the other end of the clamping arm (122) from pivoting freely;
wherein, when the sleeve portion (81) is clamped in the groove (110), the ring portion (82) abuts against the top end of the handle (123); and
wherein, in the disassembly process of the first connection assembly (1) from the second connection assembly (2), the ring portion (82) can be moved, so that the sleeve portion (81) can move along the sleeve member (11) and detach from the groove (110); as the handle (123) is no longer blocked by the sleeve portion (81) and can be pressed, the other end of the clamp arm (122) can freely pivot.

6. The closed connector according to claim 3, wherein the second sleeve (40) comprises a stabilizing structure (44), and the stabilizing structure (44) is located below the protrusion (43); after the second sleeve (40) enters the first sleeve (10), the stabilizing structure (44) abuts against the inner side wall of the first sleeve (10).

7. The closed connector according to claim 1, wherein the second sleeve (40) has two threaded portions (411, 422), and one of the threaded portions (422) defines a second opening (423), has an external thread and is adapted for screwing on a syringe, and the other threaded portion (411) has an internal thread and is adapted for screwing on the medicine bottle (200) or the human body catheter.

## Patentansprüche

1. Geschlossener Verbinder mit komprimierbarer Stoßverbindung zur Absorption von flüssigen Medikamentenresten bei der Demontage, umfassend:
eine erste Verbindungsbaugruppe (1), bestehend aus:
eine erste Hülse (10), folgendes umfasst: ein erstes Rohr (13) und eine Positionierungsrille (14), wobei das erste Rohr (13) eine erste Öffnung (131) definiert;
ein Kupplungselement (20) mit einem ersten Ende (21) und einem zweiten Ende (22), wobei das erste Ende (21) des Kupplungselements (20) innerhalb der ersten Hülse (10) angeordnet ist und das zweite Ende (22) des Kupplungs-elements (20) zum Aufschrauben einer Spritze (100) oder eines Infusions-schlauchs ausgebildet ist; und
einen ersten elastischen Körper (30), umfassend: einen Umfangsabschnitt (31) und einen Dichtungsabschnitt (32); wobei der Umfangsabschnitt (31) in der Positionierungsrille (14) positioniert ist und sich in das erste Rohr (13) erstreckt; wobei das erste Ende (21) des Kupplungselements (20) auf das obere Ende des Umfangsabschnitts (31) des ersten elastischen Körpers (30) drückt; wobei der Dichtungsabschnitt (32) in dem ersten Rohr (13) vorgesehen ist, um die erste Öffnung (131) zu schließen; und
eine zweite Verbindungsbaugruppe (2), einschließlich:
eine zweite Hülse (40) mit einer zweiten Öffnung (423), die zur lösbaren Verbindung mit einer Medikamentenflasche (200) oder einem mensch-lichen Körperkatheter geeignet ist; und
ein Verbindungsrohr (50), das in der zweiten Hülse (40) angeordnet ist, ein geschlossenes oberes Ende (511) aufweist und mit mindestens einer Perforation (512) versehen ist;
**dadurch gekennzeichnet, daß** das Kupplungselement (20) einen Blockierabschnitt (23) aufweist, und der Blockierabschnitt (23) an der Innenseite des ersten Endes (21) des Kupplungselements (20) vorgesehen ist; der Umfangsabschnitt (31) einen Aufnahmeraum (311) und mindestens ein Ansaugloch (313) aufweist; der Aufnahmeraum (311) das obere Ende des Umfangsabschnitts (31) durchdringt, wobei das mindestens eine Ansaugloch (313) zwischen dem Aufnahmeraum (311) und der Innenseite des ersten Rohrs (13) verbunden ist; die Innenseite des ersten Rohrs (13) mindestens eine Stützrippe (132) aufweist, daß eine äußere Seitenwand des ersten elastischen Körpers gegen die mindestens eine Stützrippe (132) stößt, daß der Blockierabschnitt (23) des Kupplungselements gegen das obere Ende des Umfangsabschnitts (31) stößt und eine Öffnung (314) des Aufnahmeraums (311) verschließt;
daß während eines Andockvorgangs der ersten Verbindungsbaugruppe (1) und der zweiten Verbindungsbaugruppe (2) die Verbindungsröhre (50) auf die zweite Verbindungsbaugruppe drückt und komprimiert und der Dichtungsabschnitt (32) komprimiert den Umfangsabschnitt (31), so daß die Luft in dem Aufnahmeraum (311) durch das mindestens eine Ansaugloch (313) in das Innere der ersten Röhre (13) eintritt, und das mindestens eine Ansaugloch (313) verformt und geschlossen wird, um den Aufnahmeraum (311) in einen Vakuumzustand zu versetzen; das Verbindungsrohr (50) durch die erste Öffnung (131) in das Innere des ersten Rohrs (13) führt, und die mindestens eine Perforation (512) in dem ersten Rohr (13) angeordnet ist, so daß das Innere der ersten Hülse (10) mit dem Inneren der zweiten Hülse (40) in Verbindung steht;
daß während eines Demontageprozesses der ersten Verbindungsbaugruppe (1) und der zweiten Verbindungsbaugruppe (2) das Verbindungsrohr (50) von dem ersten Rohr (13) getrennt wird; und
daß in einem Prozess der Trennung der Verbindungsröhre (50) von der ersten Röhre (13) die erste Röhre (13) die äußere Oberfläche der Verbindungsröhre (50) berührt, und in einem Prozess der Wiederherstellung der ursprünglichen Form des ersten elastischen Körpers (30) durch elastische Kraft der Umfangsabschnitt (31) wiederhergestellt wird, indem er mit elastischer Kraft gegen den Dichtungs-abschnitt (32) gedrückt wird, um die erste Öffnung (131) zu schließen, und das mindestens eine Ansaugloch (313) wieder in seine offene Position gebracht wird, die Luft im Inneren der ersten Röhre (13) durch das mindestens eine Ansaugloch (313) in den Aufnahmeraum (311) eintritt, um einen Unterdruck zu erzeugen, und die flüssige Medizin auf der Außenfläche der Verbindungsröhre (50) ver-anlasst wird, durch eine Schabwirkung der ersten Röhre (13) und eine Ansaug-wirkung des Unterdrucks in die erste Röhre (13) einzutreten, und die flüssige Medizin im Inneren der ersten Röhre (13) weiter durch das mindestens eine Ansaugloch (313) hindurchgeht, um durch die Ansaugwirkung des Unterdrucks in dem Aufnahmeraum (311) gesammelt zu werden.

2. Geschlossener Verbinder nach Anspruch 1, wobei das erste Rohr (13A) ein unteres Ende aufweist, das einen Eingriffsabschnitt (133) bildet, und das erste Rohr (13A) mit einem geschlossenen Kragen (134) versehen ist, wobei der geschlossene Kragen (134) aus weichem Material besteht und mit der ersten Öffnung (131A) und einer an der Außenseite ausgesparten Nut (135) angeordnet ist; der Eingriffsabschnitt (133) in der Nut (135) festgeklemmt ist und der Dichtungsabschnitt (32A) in dem geschlossenen Kragen (134) vorgesehen ist;
wobei der erste elastische Körper (30A) einen ersten Schnallenabschnitt (34) aufweist, der erste Schnallenabschnitt (34) auf dem Dichtungsabschnitt (32A) angeordnet ist und der erste Schnallenabschnitt (34) ein ausgespartes Loch (341) und eine Kerbe (342) umfasst; wobei das ausgesparte Loch (341) im Inneren des Dichtungsabschnitts (32A) angeordnet ist, die Kerbe (342) am unteren Ende der Dichtungsabschnitt (32A), und der Durchmesser der Kerbe (342) ist kleiner als der maximale Durchmesser des ausgesparten Lochs (341);
wobei das Verbindungsrohr (50A) einen zweiten Schnallenabschnitt (54) umfasst, der zweite Schnallenabschnitt (54) einen Hals (541) und einen Kopf (542) umfasst; der Hals (541) am geschlossenen Ende (511) vorstehend angeordnet ist, der Kopf (542) an einem Ende des Halses (541) angeordnet ist, der Durchmesser des Halses (541) kleiner ist als der maximale Durchmesser des Kopfes (542), die Größe und Form des Kopfes (542) der Größe und Form des ausgesparten Loches (341) entsprechen und die Größe und Form des Halses (541) der Größe und Form der Kerbe (342) entsprechen;
wobei bei einem Andockvorgang der ersten Verbindungsbaugruppe (1A) und der zweiten Verbindungsbaugruppe (2A) der Kopf (542) in das ausgesparte Loch (341) eintritt und der Hals (541) in der Kerbe (342) angeordnet ist, so daß der zweite Schnallenabschnitt (54) an den ersten Schnallenabschnitt (34) geknickt wird; der Dichtungsabchnitt (32A) von dem geschlossenen Kragen (134) getrennt wird, das Verbindungsrohr (50A) durch den geschlossenen Kragen (134) in das erste Rohr (13A) tritt;
wobei in einem Demontageprozess der ersten Verbindungsbaugruppe (1A) und der zweiten Verbindungsbaugruppe (2A) das Verbindungsrohr (50A) von dem geschlossenen Kragen (134) getrennt wird und schließlich der zweite Schnallen-abschnitt (54) aus dem ersten Schnallenabschnitt (34) herausgezogen wird, so daß der erste Schnallenabschnitt (34) und der zweite Schnallenabschnitt (54) getrennt werden; und
wobei in einem Prozess des Trennens des Verbindungsrohrs (50A) von dem geschlos-senen Kragen (134) der geschlossene Kragen (134) die äußere Oberfläche des Verbindungsrohrs (50A) berührt, in einem Prozess der Wiederherstellung der ursprünglichen Form des ersten elastischen Körpers (30A) durch elastische Kraft der Dichtungsabschnitt (32A) in den geschlossenen Kragen (134) eindringt und die flüssige Medizin auf der äußeren Oberfläche des Verbindungsrohrs (50A) durch die Abstreifwirkung des geschlossenen Kragens (134) in das erste Rohr (13A) eindringt.

3. Geschlossener Verbinder nach Anspruch 1 oder 2, wobei die erste Hülse (10) min-destens einen Klemmabschnitt (12) umfasst und die zweite Hülse (40) einen Vorsprung (43) umfasst;
wobei während des Andockvorgangs der ersten Verbindungsbaugruppe (1) und der zweiten Verbindungsbaugruppe (2) der mindestens eine Klemmabschnitt (12) den Vorsprung (43) festklemmt;
wobei durch den Vorsprung (43), der sich willkürlich in einer ersten Richtung oder einer zweiten Richtung entlang des mindestens einen Klemmabschnitts (12) dreht, die erste Verbindungsbaugruppe (1) sich entlang der zweiten Verbindungs-baugruppe (2) zu bewegen, um sich beliebig in die erste Richtung und die zweite Richtung zu drehen; und
wobei während des Demontageprozesses der ersten Verbindungsbaugruppe (1) und der zweiten Verbindungsbaugruppe (2) der mindestens eine Klemmabschnitt (12) von dem Vorsprung (43) getrennt wird.

4. Geschlossener Verbinder nach Anspruch 3, wobei die erste Hülse (10) ein Hülsen-element (11) umfasst, und das Hülsenelement (11) mindestens eine Perforation (111) definiert, wobei die mindestens eine Perforation (111) eine Seitenwand des Hülsen-elements (11) durchdringt und mit der Innenseite des Hülsenelements (11) in Verbindung steht; der mindestens eine Klemmabschnitt (12) einen Verbindungs-abschnitt (121) und einen Klemmarm (122) umfasst, wobei der Verbindungsabschnitt (121) an der Außenseite des Hülsenelements (11) vorstehend angeordnet ist und sich oberhalb der mindestens einen Perforation (111) befindet, und der Klemmarm (122) an der Außenseite des Verbindungsabschnitts (121) drehbar gelagert ist, ein Ende des Klemmarms (122) ein Griff (123) ist, und das andere Ende des Klemmarms (122) einen Haken (125) bildet, und der Haken (125) an der mindestens einen Perforation (111) angeordnet ist; der Vorsprung (43) ist um die Außenseite der zweiten Hülse (40) herum angeordnet;
wobei beim Andockvorgang der ersten Verbindungsbaugruppe (1) und der zweiten Verbindungsbaugruppe (2) der Vorsprung (43) sich in die mindestens eine Perforation (111) bewegt und der Haken (125) das untere Ende des Vorsprungs (43) einhakt;
wobei durch den Vorsprung (43), der willkürlich in der ersten Richtung und der zweiten Richtung entlang des Hakens (125) gedreht werden kann, die erste Verbindungs-baugruppe (1) willkürlich entlang der ersten Richtung und der zweiten Richtung relativ zu der zweiten Verbindungsanordnung (2) gedreht werden kann; und
wobei bei der Demontage der ersten Verbindungsbaugruppe (1) und der zweiten Verbindungsbaugruppe (2) durch Drücken des Griffs (123) das andere Ende des Klemmarms (122) nach außen schwenkt und der Haken (125) von dem Vorsprung (43) gelöst wird.

5. Geschlossener Verbinder nach Anspruch 4, wobei eine Nut (110) zwischen dem Griff (123) und dem Hülsenelement (11) ausgebildet ist und der geschlossene Verbinder ferner einen Sicherheitsring (80) umfasst, und der Sicherheitsring (80) einen Hülsen-abschnitt (81) und einen Ringabschnitt (82) umfasst; wobei der Hülsenabschnitt (81) beweglich auf das Hülsenelement (11) geschoben ist und der Ringabschnitt (82) am oberen Ende des Hülsenabschnitts (81) angeordnet ist;
wobei, nachdem die erste Verbindungsbaugruppe (1) und die zweite Verbindungs-baugruppe (2) im Gebrauch angedockt sind, der Hülsenabschnitt (81) sich entlang des Hülsenelements (11) in die Nut (110) bewegt, so daß der Hülsen-abschnitt (81) in der Nut (110) festgeklemmt wird, um den Griff (123) und verhindern, daß das andere Ende des Klemmarms (122) frei schwenken kann;
wobei, wenn der Hülsenabschnitt (81) in der Nut (110) festgeklemmt ist, der Ring-abschnitt (82) gegen das obere Ende des Griffs (123) stößt; und
wobei beim Demontagevorgang der ersten Verbindungsbaugruppe (1) von der zweiten Verbindungsbaugruppe (2) der Ringabschnitt (82) bewegt werden kann, so daß sich der Hülsenabschnitt (81) entlang des Hülsenelements (11) bewegen und aus der Nut (110) lösen kann; da der Griff (123) nicht mehr durch den Hülsen-abschnitt (81) blockiert ist und gedrückt werden kann, kann das andere Ende des Klemmarms (122) frei schwenken.

6. Geschlossener Verbinder nach Anspruch 3, wobei die zweite Hülse (40) eine stabili-sierende Struktur (44) aufweist und die stabilisierende Struktur (44) unterhalb des Vorsprungs (43) angeordnet ist; nachdem die zweite Hülse (40) in die erste Hülse (10) eingetreten ist, stößt die stabilisierende Struktur (44) gegen die innere Seitenwand der ersten Hülse (10).

7. Geschlossener Verbinder nach Anspruch 1, wobei die zweite Hülse (40) zwei Gewindeabschnitte (411, 422) aufweist und einer der Gewindeabschnitte (422) eine zweite Öffnung (423) definiert, ein Außengewinde aufweist und zum Aufschrauben auf eine Spritze geeignet ist, und der andere Gewindeabschnitt (411) ein Innengewinde aufweist und zum Aufschrauben auf die Medikamentenflasche (200) oder den mensch-lichen Körperkatheter geeignet ist.

## Revendications

1. Connecteur fermé avec joint d'étanchéité compressible pour absorber les résidus de médicaments liquides lors du démontage, comprenant :
un premier ensemble de connexion (1), comprenant :
un premier manchon (10), comprenant : un premier tube (13) et une rainure de positionnement (14), le premier tube (13) définissant une première ouverture (131) ;
un élément d'accouplement (20) ayant une première extrémité (21) et une seconde extrémité (22), la première extrémité (21) de l'élément d' accouplement (20) étant disposée à l'intérieur du premier manchon (10), la seconde extrémité (22) de l'élément d'accouplement (20) étant conçue pour le vissage d'une seringue (100) ou d'un tube de perfusion ; et
un premier corps élastique (30), comprenant : une partie circonférentielle (31) et une partie d'étanchéité (32) ; la partie circonférentielle (31) étant positionnée dans la rainure de positionnement (14) et s'étendant dans le premier tube (13) ; la première extrémité (21) de l'élément d'accouplement (20) appuyant sur l'extrémité supérieure de la partie circonférentielle (31) du premier corps élastique (30) ; la partie d'étanchéité (32) étant prévue dans le premier tube (13) pour fermer la première ouverture (131) ; et
un second ensemble de connexion (2), comprenant :
un second manchon (40) doté d'une seconde ouverture (423) et conçu pour se connecter de manière amovible à un flacon de médicaments (200) ou à un cathéter pour le corps humain ; et
un tube de connexion (50), disposé dans le second manchon (40), ayant une extrémité supérieure fermée (511) et comportant au moins une perforation (512) ;
**caractérisé en ce que** l'élément d'accouplement (20) a une partie de blocage (23), et la partie de blocage (23) est prévue à l'intérieur de la première extrémité (21) de l'élément d'accouplement (20) ; la partie circonférentielle (31) présente un espace de logement (311) et au moins un trou d'aspiration (313) ; l'espace de logement (311) traverse l'extrémité supérieure de la partie circonférentielle (31), l'au moins un trou d'aspiration (313) étant relié entre l'espace de logement (311) et l'intérieur du premier tube (13) ; l'intérieur du premier tube (13) comporte au moins une nervure de support (132), une paroi latérale extérieure du premier corps élastique venant en butée contre l'au moins une nervure de support (132), la partie de blocage (23) de l'élément d'accouplement venant en butée contre l'extrémité supérieure de la partie circonférentielle (31) et fermant une ouverture (314) de l'espace de logement (311) ;
en ce sens que, pendant le processus d'accostage du premier ensemble de connexion (1) et du second ensemble de connexion (2), le tube de connexion (50) pousse et comprime la partie d'étanchéité (32), et la partie d'étanchéité (32) comprime la partie circonférentielle (31), de sorte que l'air dans l'espace de logement (311) pénètre à l'intérieur du premier tube (13) à travers ledit au moins un trou d'aspiration (313), et ledit au moins un trou d'aspiration (313) est déformé et fermé pour rendre l'espace de logement (311) dans un état de vide ; le tube de connexion (50) passe à travers la première ouverture (131) à l'intérieur du premier tube (13), et l'au moins une perforation (512) est située dans le premier tube (13), de sorte que l'intérieur du premier manchon (10) communique avec l'intérieur du second manchon (40) ;
le tube de connexion (50) est séparé du premier tube (13) au cours du processus de démontage du premier ensemble de connexion (1) et du second ensemble de connexion (2) ; et
en effet, lors de la séparation du tube de connexion (50) du premier tube (13), le premier tube (13) entre en contact avec la surface extérieure du tube de connexion (50), et lors du rétablissement de la forme originale du premier corps élastique (30) par une force élastique, la partie circonférentielle (31) est rétablie en poussant contre la partie d'étanchéité (32) avec une force élastique pour fermer la première ouverture (131), et ledit au moins un trou d'aspiration (313) est rétabli dans sa position d'ouverture, l'air à l'intérieur du premier tube (13) pénètre dans l'espace de logement (311) par le biais d'au moins un trou d'aspiration (313) pour générer une pression négative, et le médicament liquide sur la surface extérieure du tube de connexion (50) est amené à pénétrer dans le premier tube (13) par une action de raclage du premier tube (13) et une action d'aspiration de la pression négative, et le médicament liquide à l'intérieur du premier tube (13) passe ensuite par le biais d'au moins un trou d'aspiration (313) pour être collecté dans l'espace de logement (311) par l'action d'aspiration de la pression négative.

2. Connecteur fermé selon la revendication 1, dans lequel le premier tube (13A) a une extrémité inférieure qui forme une partie d'engagement (133), et le premier tube (13A) est pourvu d'un collier fermé (134), le collier fermé (134) étant fait d'un matériau souple et disposé avec la première ouverture (131A) et une rainure (135) encastrée sur le côté extérieur ; la partie d'engagement (133) est serrée dans la rainure (135), et la partie d'étanchéité (32A) est prévue dans le collier fermé (134) ;
dans lequel, le premier corps élastique (30A) a une première partie de boucle (34), la première partie de boucle (34) est disposée sur la partie d'étanchéité (32A), et la première partie de boucle (34) comprend un trou en retrait (341) et une encoche (342) ; le trou en retrait (341) est situé à l'intérieur de la partie d'étanchéité (32A), l'encoche (342) est située à l'extrémité inférieure de la partie d'étanchéité (32A), et l'encoche (342) est située à l'extrémité inférieure de la partie d'étanchéité (32A), et le diamètre de l'encoche (342) est inférieur au diamètre maximal du trou en retrait (341) ;
dans lequel le tube de connexion (50A) comprend une second partie de boucle (54), la second partie de boucle (54) comprend un col (541) et une tête (542) ; le col (541) est disposé en saillie à l'extrémité fermée (511), la tête (542) est disposée à une extrémité du col (541), le diamètre du col (541) est inférieur au diamètre maximal de la tête (542), la taille et la forme de la tête (542) correspondent à la taille et à la forme du trou en retrait (341), et la taille et la forme du col (541) correspondent à la taille et à la forme de l'encoche (342) ;
dans lequel, lors d'un processus d'accostage du premier ensemble de connexion (1A) et du second ensemble de connexion (2A), la tête (542) pénètre dans le trou en retrait (341), et le col (541) est situé dans l'encoche (342), de sorte que la seconde partie de boucle (54) est bouclée à la première partie de boucle (34) ; la partie d'étanchéité (32A) est séparée du collier fermé (134), le tube communicant (50A) passe à travers le collier fermé (134) dans le premier tube (13A) ;
dans lequel, lors d'un processus de démontage du premier ensemble de connexion (1A) et du second ensemble de connexion (2A), le tube de connexion (50A) est séparé du collier fermé (134), et enfin la seconde partie de boucle (54) est retirée de la première partie de boucle (34), de sorte que la première partie de boucle (34) et la seconde partie de boucle (54) sont séparées ; et
dans lequel, lors de la séparation du tube de connexion (50A) du collier fermé (134), le collier fermé (134) entre en contact avec la surface extérieure du tube de connexion (50A), lors du rétablissement de la forme originale du premier corps élastique (30A) sous l'effet de la force élastique, la partie étanche (32A) pénètre dans le collier fermé (134), et le médicament liquide sur la surface extérieure du tube de connexion (50A) pénètre dans le premier tube (13A) par l'effet de grattage du collier fermé (134).

3. Connecteur fermé selon les revendications 1 ou 2, dans lequel le premier manchon (10) comprend au moins une partie de serrage (12), et le second manchon (40) comprend une protubérance (43) ;
dans lequel, pendant le processus d'accostage du premier ensemble de connexion (1) et du second ensemble de connexion (2), au moins une partie de serrage (12) serre la protubérance (43) ;
dans lequel, par la rotation arbitraire de la protubérance (43) dans une première direction ou une seconde direction le long de la partie de serrage (12) au moins, le premier ensemble de connexion (1) peut se déplacer le long du second ensemble de connexion (2) pour tourner arbitrairement dans la première direction et la seconde direction ; et
dans lequel, au cours du processus de démontage du premier ensemble de connexion (1) et du second ensemble de connexion (2), l'au moins une partie de serrage (12) est séparée de la protubérance (43).

4. Connecteur fermé selon la revendication 3, dans lequel le premier manchon (10) comprend un élément de manchon (11), et l'élément de manchon (11) définit au moins une perforation (111), ledit au moins une perforation (111) pénètre dans une paroi latérale de l'élément de manchon (11) et communique avec l'intérieur de l'élément de manchon (11) ; ledit au moins une partie de serrage (12) comprend une partie de connexion (121) et un bras de serrage (122), la partie de connexion (121) est disposée en saillie sur l'extérieur de l'élément de manchon (11) et située au-dessus de l'au moins une perforation (111), et le bras de serrage (122) est pivoté sur l'extérieur de la partie de connexion (121), une extrémité du bras de serrage (122) est une poignée (123), et l'autre extrémité du bras de serrage (122) forme un crochet (125), et le crochet (125) est situé au niveau de l'au moins une perforation (111) ; la protubérance (43) est disposée autour de l'extérieur du second manchon (40) ;
dans lequel, lors du processus d'accostage du premier ensemble de connexion (1) et du second ensemble de connexion (2), la protubérance (43) se déplace dans l'au moins une perforation (111), et le crochet (125) accroche l'extrémité inférieure de la protubérance (43) ;
dans lequel, en faisant tourner arbitrairement la protubérance (43) dans la première direction et la seconde direction le long du crochet (125), le premier ensemble de connexion (1) peut être tourné arbitrairement dans la première direction et la seconde direction par rapport au second ensemble de connexion (2) ; et
dans lequel, au cours du processus de démontage du premier ensemble de connexion (1) et du second ensemble de connexion (2), en appuyant sur la poignée (123), l'autre extrémité du bras de serrage (122) pivote vers l'extérieur, le crochet (125) est détaché de la protubérance (43).

5. Connecteur fermé selon la revendication 4, dans lequel une rainure (110) est formée entre la poignée (123) et l'élément de manchon (11), et le connecteur fermé comprend en outre une bague de sécurité (80), et la bague de sécurité (80) comprend une partie manchon (81) et une partie annulaire (82) ; la partie manchon (81) est manchonnée de manière mobile sur l'élément de manchon (11), et la partie annulaire (82) est disposée à l'extrémité supérieure de la partie manchon (81) ;
dans lequel, après que le premier ensemble de connexion (1) et le second ensemble de connexion (2) ont été accostés en cours d'utilisation, la partie manchon (81) se déplace le long de l'élément de manchon (11) dans la rainure (110) de sorte que la partie manchon (81) est serrée dans la rainure (110) pour bloquer la poignée (123) et empêcher l'autre extrémité du bras de serrage (122) de pivoter librement;
dans lequel, lorsque la partie manchon (81) est serrée dans la rainure (110), la partie annulaire (82) vient en butée contre l'extrémité supérieure de la poignée (123) ; et
dans lequel, lors du processus de démontage du premier ensemble de connexion (1) du second ensemble de connexion (2), la partie annulaire (82) peut être déplacée, de sorte que la partie manchon (81) peut se déplacer le long de l'élément de manchon (11) et se détacher de la rainure (110) ; comme la poignée (123) n'est plus bloquée par la partie manchon (81) et peut être pressée, l'autre extrémité du bras de serrage (122) peut pivoter librement.

6. Connecteur fermé selon la revendication 3, dans lequel le second manchon (40) comprend une structure de stabilisation (44), et la structure de stabilisation (44) est située sous la protubérance (43) ; après que le second manchon (40) a pénétré dans le premier manchon (10), la structure de stabilisation (44) vient en butée contre la paroi latérale intérieure du premier manchon (10).

7. Connecteur fermé selon la revendication 1, dans lequel le second manchon (40) a deux parties filetées (411, 422), et l'une des parties filetées (422) définit une seconde ouverture (423), a un filetage externe et est conçue pour être vissée sur une seringue, et l'autre partie filetée (411) a un filetage interne et est conçue pour être vissée sur le flacon de médicaments (200) ou le cathéter du corps humain.
